# EUROPEAN PATENT APPLICATION

(11) **EP 3 210 631 A1**
(43) Date of publication of application: **30.08.2017**
(21) Application number: 15852539.4
(22) Date of filing: 16.06.2015
(51) Int. Cl.: A61K 48/00, A61P 31/22

(54) **DNA VACCINE COMPOSITION FOR PREVENTING AND TREATING HERPES ZOSTER, AND METHOD FOR ACTIVATING T CELLS FOR VZV ANTIGEN BY USING SAME**

(30) Priority: 21.10.2014 KR 20140142531
(71) Applicant: Geneone Life Science Co., Ltd., Seoul 135-914 (KR)
(72) Inventor: PARK, Young Kun, Wyndrise, Pennsylvania 19422 (US); CHO, Byung Mun, Ganghwa-gun Incheon 417-841 (KR); JEONG, Moon Sup, Incheon 406-840 (KR); LEE, Hyo Jin, Seongnam-si Gyeonggi-do 463-826 (KR); CHO, Young Ran, Seoul 122-942 (KR); OH, Joo Yeon, Seoul 158-777 (KR)
(74) Representative: Ter Meer Steinmeister & Partner
(86) International application number: PCT/KR2015/006043
(87) International publication number: WO 2016/064063

(57) **Abstract**

The present invention provides a DNA vaccine composition for preventing and treating herpes zoster, containing: at least one type of plasmid containing the insertion site of a varicella-zoster virus (VZV)-derived gene encoding a VZV protein; and other pharmaceutically acceptable ingredients. The plasmid contains a plurality of plasmids containing the insertion site of heterologous genes which are different from each other. The plasmid contains: a first plasmid containing the insertion site of a first gene encoding IE-62 protein (SEQ ID NO: 1); a second plasmid containing the insertion site of a second gene encoding IE-63 protein (SEQ ID NO: 2); and a third plasmid containing the insertion site of a third gene encoding gE protein (SEQ ID NO: 3).

<110>    GENEONE LIFE SCIENCE

<120>    DNA VACCINE COMPOSITION FOR PREVENTING AND TREATING HERPES ZOSTER
         AND METHOD OF ACTIVATING T-CELL TO VZV ANTIZEN BY USING THE SAME

<130>    KP14-001

<160>    6

<170>    KopatentIn 2.0

<210>    1
<211>    1328
<212>    PRT
<213>    Varicella-Zoster Virus

<400>    1
   Met Asp Trp Thr Trp Ile Leu Phe Leu Val Ala Ala Ala Thr Arg Val
     1               5                  10                  15
   His Ser Met Asp Thr Pro Pro Met Gln Arg Ser Thr Pro Gln Arg Ala
                20                  25                  30
   Gly Ser Pro Asp Thr Leu Glu Leu Met Asp Leu Leu Asp Ala Ala Ala
            35                  40                  45
   Ala Ala Ala Glu His Arg Ala Arg Val Val Thr Ser Ser Gln Pro Asp
        50                  55                  60
   Asp Leu Leu Phe Gly Glu Asn Gly Val Met Val Gly Arg Glu His Glu
    65                  70                  75                  80
   Ile Val Ser Ile Pro Ser Val Ser Gly Leu Gln Pro Glu Pro Arg Thr
                    85                  90                  95
   Glu Asp Val Gly Glu Glu Leu Thr Gln Asp Asp Tyr Val Cys Glu Asp
               100                 105                 110
   Gly Gln Asp Leu Met Gly Ser Pro Val Ile Pro Leu Ala Glu Val Phe
           115                 120                 125
   His Thr Arg Phe Ser Glu Ala Gly Ala Arg Glu Pro Thr Gly Ala Asp
       130                 135                 140
   Arg Ser Leu Glu Thr Val Ser Leu Gly Thr Lys Leu Ala Arg Ser Pro
   145                 150                 155                 160
   Lys Pro Pro Met Asn Asp Gly Glu Thr Gly Arg Gly Thr Thr Pro Pro
                   165                 170                 175
   Phe Pro Gln Ala Phe Ser Pro Val Ser Pro Ala Ser Pro Val Gly Asp
               180                 185                 190
   Ala Ala Gly Asn Asp Gln Arg Glu Asp Gln Arg Ser Ile Pro Arg Gln
           195                 200                 205
   Thr Thr Arg Gly Asn Ser Pro Gly Leu Pro Ser Val Val His Arg Asp
       210                 215                 220
   Arg Gln Thr Gln Ser Ile Ser Gly Lys Lys Pro Gly Asp Glu Gln Ala
   225                 230                 235                 240
   Gly His Ala His Ala Ser Gly Asp Gly Val Val Leu Gln Lys Thr Gln
   245                 250                 255
   Arg Pro Ala Gln Gly Lys Ser Pro Lys Lys Lys Thr Leu Lys Val Lys
               260                 265                 270
   Val Pro Leu Pro Ala Arg Lys Pro Gly Gly Pro Val Pro Gly Pro Val
           275                 280                 285
   Glu Gln Leu Tyr His Val Leu Ser Asp Ser Val Pro Ala Lys Gly Ala
       290                 295                 300
   Lys Ala Asp Leu Pro Phe Glu Thr Asp Asp Thr Arg Pro Arg Lys His
   305                 310                 315                 320
   Asp Ala Arg Gly Ile Thr Pro Arg Val Pro Gly Arg Ser Ser Gly Gly
                   325                 330                 335
   Lys Pro Arg Ala Phe Leu Ala Leu Pro Gly Arg Ser His Ala Pro Asp
               340                 345                 350
   Pro Ile Glu Asp Asp Ser Pro Val Glu Lys Lys Pro Lys Ser Arg Glu
           355                 360                 365
   Phe Val Ser Ser Ser Ser Ser Ser Ser Ser Trp Gly Ser Ser Ser Glu
       370                 375                 380
   Asp Glu Asp Asp Glu Pro Arg Arg Val Ser Val Gly Ser Glu Thr Thr
   385                 390                 395                 400
   Gly Ser Arg Ser Gly Arg Glu His Ala Pro Ser Pro Ser Asn Ser Asp
                   405                 410                 415
   Asp Ser Asp Ser Asn Asp Gly Gly Ser Thr Lys Gln Asn Ile Gln Pro
               420                 425                 430
   Gly Tyr Arg Ser Ile Ser Gly Pro Asp Pro Arg Ile Arg Lys Thr Lys
           435                 440                 445
   Arg Leu Ala Gly Glu Pro Gly Arg Gln Arg Gln Lys Ser Phe Ser Leu
       450                 455                 460
   Pro Arg Ser Arg Thr Pro Ile Ile Pro Pro Val Ser Gly Pro Leu Met
   465                 470                 475                 480
   Met Pro Asp Gly Ser Pro Trp Pro Gly Ser Ala Pro Leu Pro Ser Asn
                   485                 490                 495
   Arg Val Arg Phe Gly Pro Ser Gly Glu Thr Arg Glu Gly His Trp Glu
               500                 505                 510
   Asp Glu Ala Val Arg Ala Ala Arg Ala Arg Tyr Glu Ala Ser Thr Glu
           515                 520                 525
   Pro Val Pro Leu Tyr Val Pro Glu Leu Gly Asp Pro Ala Arg Gln Tyr
       530                 535                 540
   Arg Ala Leu Ile Asn Leu Ile Tyr Cys Pro Asp Arg Asp Pro Ile Ala
   545                 550                 555                 560
   Trp Leu Gln Asn Pro Lys Leu Thr Gly Val Asn Ser Ala Leu Asn Gln
                   565                 570                 575
   Phe Tyr Gln Lys Leu Leu Pro Pro Gly Arg Ala Gly Thr Ala Val Thr
   580 585 590
   Gly Ser Val Ala Ser Pro Val Pro His Val Gly Glu Ala Met Ala Thr
   595 600 605
   Gly Glu Ala Leu Trp Ala Leu Pro His Ala Ala Ala Ala Val Ala Met
   610 615 620
   Ser Arg Arg Tyr Asp Arg Ala Gln Lys His Phe Ile Leu Gln Ser Leu
   625 630 635 640
   Arg Arg Ala Phe Ala Ser Met Ala Tyr Pro Glu Ala Thr Gly Ser Ser
   645 650 655
   Pro Ala Ala Arg Ile Ser Arg Gly His Pro Ser Pro Thr Thr Pro Ala
   660 665 670
   Thr Gln Thr Pro Asp Pro Gln Pro Ser Ala Ala Ala Arg Ser Leu Ser
   675 680 685
   Val Cys Pro Pro Asp Asp Arg Leu Arg Thr Pro Arg Lys Arg Lys Ser
   690 695 700
   Gln Pro Val Glu Ser Arg Ser Leu Leu Asp Lys Ile Arg Glu Thr Pro
   705 710 715 720
   Val Ala Asp Ala Arg Val Ala Asp Asp His Val Val Ser Lys Ala Lys
   725 730 735
   Arg Arg Val Ser Glu Pro Val Thr Ile Thr Ser Gly Pro Val Val Asp
   740 745 750
   Pro Pro Ala Val Ile Thr Met Pro Leu Asp Gly Pro Ala Pro Asn Gly
   755 760 765
   Gly Phe Arg Arg Ile Pro Arg Gly Ala Leu His Thr Pro Val Pro Ser
   770 775 780
   Asp Gln Ala Arg Lys Ala Tyr Cys Thr Pro Glu Thr Ile Ala Arg Leu
   785 790 795 800
   Val Asp Asp Pro Leu Phe Pro Thr Ala Trp Arg Pro Ala Leu Ser Phe
   805 810 815
   Asp Pro Gly Ala Leu Ala Glu Ile Ala Ala Arg Arg Pro Gly Gly Gly
   820 825 830
   Asp Arg Arg Phe Gly Pro Pro Ser Gly Val Glu Ala Leu Arg Arg Arg
   835 840 845
   Cys Ala Trp Met Arg Gln Ile Pro Asp Pro Glu Asp Val Arg Leu Leu
   850 855 860
   Ile Ile Tyr Asp Pro Leu Pro Gly Glu Asp Ile Asn Gly Pro Leu Glu
   865 870 875 880
   Ser Thr Leu Ala Thr Asp Pro Gly Pro Ser Trp Ser Pro Ser Arg Gly
   885 890 895
   Gly Leu Ser Val Val Leu Ala Ala Leu Ser Asn Arg Leu Cys Leu Pro
   900 905 910
   Ser Thr His Ala Trp Ala Gly Asn Trp Thr Gly Pro Pro Asp Val Ser
   915                 920                 925
   Ala Leu Asn Ala Arg Gly Val Leu Leu Leu Ser Thr Arg Asp Leu Ala
       930                 935                 940
   Phe Ala Gly Ala Val Glu Tyr Leu Gly Ser Arg Leu Ala Ser Ala Arg
   945                 950                 955                 960
   Arg Arg Leu Leu Val Leu Asp Ala Val Ala Leu Glu Arg Trp Pro Arg
                   965                 970                 975
   Asp Gly Pro Ala Leu Ser Gln Tyr His Val Tyr Val Arg Ala Pro Ala
               980                 985                 990
   Arg Pro Asp Ala Gln Ala Val Val Arg Trp Pro Asp Ser Ala Val Thr
           995                 1000                1005
   Glu Gly Leu Ala Arg Ala Val Phe Ala Ser Ser Arg Thr Phe Gly Pro
       1010                1015                1020
   Ala Ser Phe Ala Arg Ile Glu Thr Ala Phe Ala Asn Leu Tyr Pro Gly
   1025                1030                1035                1040
   Glu Gln Pro Leu Cys Leu Cys Arg Gly Gly Asn Val Ala Tyr Thr Val
                   1045                1050                1055
   Cys Thr Arg Ala Gly Pro Lys Thr Arg Val Pro Leu Ser Pro Arg Glu
               1060                1065                1070
   Tyr Arg Gln Tyr Val Leu Pro Gly Phe Asp Gly Cys Lys Asp Leu Ala
           1075                1080                1085
   Arg Gln Ser Arg Gly Leu Gly Leu Gly Ala Ala Asp Phe Val Asp Glu
       1090                1095                1100
   Ala Ala His Ser His Arg Ala Ala Asn Arg Trp Gly Leu Gly Ala Ala
   1105                1110                1115                1120
   Leu Arg Pro Val Phe Leu Pro Glu Gly Arg Arg Pro Gly Ala Ala Gly
                   1125                1130                1135
   Pro Glu Ala Gly Asp Val Pro Thr Trp Ala Arg Val Phe Cys Arg His
               1140                1145                1150
   Ala Leu Leu Glu Pro Asp Pro Ala Ala Glu Pro Leu Val Leu Pro Pro
           1155                1160                1165
   Val Ala Gly Arg Ser Val Ala Leu Tyr Ala Ser Ala Asp Glu Ala Arg
       1170                1175                1180
   Asn Ala Leu Pro Pro Ile Pro Arg Val Met Trp Pro Pro Gly Phe Gly
   1185                1190                1195                1200
   Ala Ala Glu Thr Val Leu Glu Gly Ser Asp Gly Thr Arg Phe Val Phe
                   1205                1210                1215
   Gly His His Gly Gly Ser Glu Arg Pro Ala Glu Thr Gln Ala Gly Arg
               1220                1225                1230
   Gln Arg Arg Thr Ala Asp Asp Arg Glu His Ala Leu Glu Pro Asp Asp
           1235                1240                1245
   Trp Glu Val Gly Cys Glu Asp Ala Trp Asp Ser Glu Glu Gly Gly Gly
   1250                    1255                    1260
   Asp Asp Gly Asp Ala Pro Gly Ser Ser Phe Gly Val Ser Ile Val Ser
   1265                1270                1275                1280
   Val Ala Pro Gly Val Leu Arg Asp Arg Arg Val Gly Leu Arg Pro Ala
                   1285                1290                1295
   Val Lys Val Glu Leu Leu Ser Ser Ser Ser Ser Ser Glu Asp Glu Asp
               1300                1305                1310
   Asp Val Trp Gly Gly Arg Gly Gly Arg Ser Pro Pro Gln Ser Arg Gly
           1315                1320                1325

<210>    2
<211>    296
<212>    PRT
<213>    Varicella-Zoster Virus

<400>    2
Met Asp Trp Thr Trp Ile Leu Phe Leu Val Ala Ala Ala Thr Arg Val
1               5                   10                  15

His Ser Met Phe Cys Thr Ser Pro Ala Thr Arg Gly Asp Ser Ser Glu
            20                  25                  30

Ser Lys Pro Gly Ala Ser Val Asp Val Asn Gly Lys Met Glu Tyr Gly
        35                  40                  45

Ser Ala Pro Gly Pro Leu Asn Gly Arg Asp Thr Ser Arg Gly Pro Gly
    50                  55                  60

Ala Phe Cys Thr Pro Gly Trp Glu Ile His Pro Ala Arg Leu Val Glu
65                  70                  75                  80

Asp Ile Asn Arg Val Phe Leu Cys Ile Ala Gln Ser Ser Gly Arg Val
                85                  90                  95

Thr Arg Asp Ser Arg Arg Leu Arg Arg Ile Cys Leu Asp Phe Tyr Leu
            100                 105                 110

Met Gly Arg Thr Arg Gln Arg Pro Thr Leu Ala Cys Trp Glu Glu Leu
        115                 120                 125

Leu Gln Leu Gln Pro Thr Gln Thr Gln Cys Leu Arg Ala Thr Leu Met
    130                 135                 140

Glu Val Ser His Arg Pro Pro Arg Gly Glu Asp Gly Phe Ile Glu Ala
145                 150                 155                 160

Pro Asn Val Pro Leu His Arg Ser Ala Leu Glu Cys Asp Val Ser Asp
                165                 170                 175

Asp Gly Gly Glu Asp Asp Ser Asp Asp Asp Gly Ser Thr Pro Ser Asp
            180                 185                 190

Val Ile Glu Phe Arg Asp Ser Asp Ala Glu Ser Ser Asp Gly Glu Asp
        195                 200                 205

Phe Ile Val Glu Glu Glu Ser Glu Glu Ser Thr Asp Ser Cys Glu Pro

210                 215                 220

Asp Gly Val Pro Gly Asp Cys Tyr Arg Asp Gly Asp Gly Cys Asn Thr
225                 230                 235                 240

Pro Ser Pro Lys Arg Pro Gln Arg Ala Ile Glu Arg Tyr Ala Gly Ala
                245                 250                 255

Glu Thr Ala Glu Tyr Thr Ala Ala Lys Ala Leu Thr Ala Leu Gly Glu
            260                 265                 270

Gly Gly Val Asp Trp Lys Arg Arg Arg His Glu Ala Pro Arg Arg His
        275                 280                 285

Asp Ile Pro Pro Pro His Gly Val
    290                 295


<210>    3
<211>    641
<212>    PRT
<213>    Varicella-Zoster Virus

<400>    3
Met Asp Trp Thr Trp Ile Leu Phe Leu Val Ala Ala Ala Thr Arg Val
 1               5                  10                  15

His Ser Met Gly Thr Val Asn Lys Pro Val Val Gly Val Leu Met Gly
            20                  25                  30

Phe Gly Ile Ile Thr Gly Thr Leu Arg Ile Thr Asn Pro Val Arg Ala
        35                  40                  45

Ser Val Leu Arg Tyr Asp Asp Phe His Ile Asp Glu Asp Lys Leu Asp
    50                  55                  60

Thr Asn Ser Val Tyr Glu Pro Tyr Tyr His Ser Asp His Ala Glu Ser
65                  70                  75                  80

Ser Trp Val Asn Arg Gly Glu Ser Ser Arg Lys Ala Tyr Asp His Asn
                85                  90                  95

Ser Pro Tyr Ile Trp Pro Arg Asn Asp Tyr Asp Gly Phe Leu Glu Asn
            100                 105                 110

Ala His Glu His His Gly Val Tyr Asn Gln Gly Arg Gly Ile Asp Ser
        115                 120                 125

Gly Glu Arg Leu Met Gln Pro Thr Gln Met Ser Ala Gln Glu Asp Leu
    130                 135                 140

Gly Asp Asp Thr Gly Ile His Val Ile Pro Thr Leu Asn Gly Asp Asp
145                 150                 155                 160

Arg His Lys Ile Val Asn Val Asp Gln Arg Gln Tyr Gly Asp Val Phe
                165                 170                 175

Lys Gly Asp Leu Asn Pro Lys Pro Gln Gly Gln Arg Leu Ile Glu Val
            180                 185                 190

Ser Val Glu Glu Asn His Pro Phe Thr Leu Arg Ala Pro Ile Gln Arg
        195                 200                 205

Ile Tyr Gly Val Arg Tyr Thr Glu Thr Trp Ser Phe Leu Pro Ser Leu
    210                 215                 220

Thr Cys Thr Gly Asp Ala Ala Pro Ala Ile Gln His Ile Cys Leu Lys
225                 230                 235                 240

His Thr Thr Cys Phe Gln Asp Val Val Val Asp Val Asp Cys Ala Glu
                245                 250                 255

Asn Thr Lys Glu Asp Gln Leu Ala Glu Ile Ser Tyr Arg Phe Gln Gly
            260                 265                 270

Lys Lys Glu Ala Asp Gln Pro Trp Ile Val Val Asn Thr Ser Thr Leu
        275                 280                 285

Phe Asp Glu Leu Glu Leu Asp Pro Pro Glu Ile Glu Pro Gly Val Leu
    290                 295                 300

Lys Val Leu Arg Thr Glu Lys Gln Tyr Leu Gly Val Tyr Ile Trp Asn
305                 310                 315                 320

Met Arg Gly Ser Asp Gly Thr Ser Thr Tyr Ala Thr Phe Leu Val Thr
                325                 330                 335

Trp Lys Gly Asp Glu Lys Thr Arg Asn Pro Thr Pro Ala Val Thr Pro
            340                 345                 350

Gln Pro Arg Gly Ala Glu Phe His Met Trp Asn Tyr His Ser His Val
        355                 360                 365

Phe Ser Val Gly Asp Thr Phe Ser Leu Ala Met His Leu Gln Tyr Lys
    370                 375                 380

Ile His Glu Ala Pro Phe Asp Leu Leu Leu Glu Trp Leu Tyr Val Pro
385                 390                 395                 400

Ile Asp Pro Thr Cys Gln Pro Met Arg Leu Tyr Ser Thr Cys Leu Tyr
                405                 410                 415

His Pro Asn Ala Pro Gln Cys Leu Ser His Met Asn Ser Gly Cys Thr
            420                 425                 430

Phe Thr Ser Pro His Leu Ala Gln Arg Val Ala Ser Thr Val Tyr Gln
        435                 440                 445

Asn Cys Glu His Ala Asp Asn Tyr Thr Ala Tyr Cys Leu Gly Ile Ser
    450                 455                 460

His Met Glu Pro Ser Phe Gly Leu Ile Leu His Asp Gly Gly Thr Thr
465                 470                 475                 480

Leu Lys Phe Val Asp Thr Pro Glu Ser Leu Ser Gly Leu Tyr Val Phe
                485                 490                 495

Val Val Tyr Phe Asn Gly His Val Glu Ala Val Ala Tyr Thr Val Val
            500                 505                 510

Ser Thr Val Asp His Phe Val Asn Ala Ile Glu Glu Arg Gly Phe Pro
        515                 520                 525

Pro Thr Ala Gly Gln Pro Pro Ala Thr Thr Lys Pro Lys Glu Ile Thr
    530                 535                 540

Pro Val Asn Pro Gly Thr Ser Pro Leu Leu Arg Tyr Ala Ala Trp Thr
545                 550                 555                 560

Gly Gly Leu Ala Ala Val Val Leu Leu Cys Leu Val Ile Phe Leu Ile
                565                 570                 575

Cys Thr Ala Lys Arg Met Arg Val Lys Ala Tyr Arg Val Asp Lys Ser
            580                 585                 590

Pro Tyr Asn Gln Ser Met Tyr Tyr Ala Gly Leu Pro Val Asp Asp Phe
        595                 600                 605

Glu Asp Ser Glu Ser Thr Asp Thr Glu Glu Glu Phe Gly Asn Ala Ile
    610                 615                 620

Gly Gly Ser His Gly Gly Ser Ser Tyr Thr Val Tyr Ile Asp Lys Thr
625                 630                 635                 640

Arg

<210>    4
<211>    6951
<212>    DNA
<213>    Artificial Sequence

<220>
<223>    pGX-5101

<400>    4
gctgcttcgc gatgtacggg ccagatatac gcgttgacat tgattattga ctagttatta      60

atagtaatca attacggggt cattagttca tagcccatat atggagttcc gcgttacata     120

acttacggta aatggcccgc ctggctgacc gcccaacgac ccccgcccat tgacgtcaat     180

aatgacgtat gttcccatag taacgccaat agggactttc cattgacgtc aatgggtgga     240

gtatttacgg taaactgccc acttggcagt acatcaagtg tatcatatgc caagtacgcc     300

ccctattgac gtcaatgacg gtaaatggcc cgcctggcat tatgcccagt acatgacctt     360

atgggacttt cctacttggc agtacatcta cgtattagtc atcgctatta ccatggtgat     420

gcggttttgg cagtacatca atgggcgtgg atagcggttt gactcacggg gatttccaag     480

tctccacccc attgacgtca atgggagttt gttttggcac caaaatcaac gggactttcc     540

aaaatgtcgt aacaactccg ccccattgac gcaaatgggc ggtaggcgtg tacggtggga     600

ggtctatata agcagagctc tctggctaac tagagaaccc actgcttact ggcttatcga     660

aattaatacg actcactata gggagaccca agctggctag cgtttaaact taagcttggt     720

accgagctcg gatccgccac catggactgg acctggattc tgttcctggt ggccgctgcc     780

acacgggtgc acagcatgga tacccccccc atgcagagaa gcacccctca gagagccggc     840

agccccgaca ccctggaact gatggatctg ctggatgccg ccgctgctgc cgccgaacat     900

agagccagag tggtcaccag cagccagccc gacgatctgc tgttcggcga gaacggcgtg     960

atggtcggaa gagaacacga gatcgtgtcc atccccagcg tgtccggact gcagcctgag 1020

cccagaacag aggacgtggg cgaggaactg acccaggacg actacgtgtg cgaggacggc 1080

caggatctga tgggcagccc tgtgattccc ctggccgagg tgttccacac cagattcagc 1140

gaggcaggcg ccagagagcc cacaggcgcc gatagaagcc tggaaaccgt gtccctgggc 1200

accaagctgg ccagatcccc caagcccccc atgaacgatg gcgagacagg cagaggcacc 1260

accccaccat tccctcaggc cttctctcct gtgtcccctg ccagcccagt gggcgacgct 1320

gccggaaacg atcagagaga ggaccagcgg agcatcccca gacagaccac cagaggcaat 1380

agccctggcc tgcctagcgt ggtgcaccgg gatagacaga cccagagcat cagcggcaag 1440

aagccaggcg acgaacaggc cggacatgcc catgctagcg gagatggcgt ggtgctgcag 1500

aaaacccaga ggcctgccca gggcaagagc cccaagaaaa agaccctgaa agtgaaggtg 1560

cccctgcccg ccagaaagcc tggtggacct gtgcctggac ccgtggaaca gctgtatcac 1620

gtgctgagcg acagcgtgcc agccaagggc gccaaggccg atctgccctt cgagacagac 1680

gacacccggc ccagaaagca cgacgccaga ggcatcacac ccagagtgcc aggcagaagc 1740

tccggcggca agcctagagc ctttctggcc ctgcccggaa gaagccacgc ccccgatcct 1800

atcgaggacg acagccccgt ggaaaagaag cccaagagcc gcgagttcgt gtcctccagc 1860

agcagctcta gcagctgggg cagctccagc gaggacgagg acgatgagcc tagaagagtg 1920

tccgtgggca gcgagacaac cggcagcaga tctggcagag agcacgcccc cagccccagc 1980

aactccgacg acagcgatag caatgacggc ggctccacca agcagaacat ccagcctggc 2040

taccggtcca tctccggccc tgacccccgg atcagaaaga ccaagagact ggccggcgag 2100

cccggcagac agagacagaa gtccttcagc ctgccccggt ccagaacccc catcatccct 2160

ccagtgtccg gccctctgat gatgcccgat ggctctcctt ggcctggcag cgcccctctg 2220

ccctccaata gagtcagatt cggccccagc ggcgagacaa gagagggaca ttgggaggac 2280

gaagccgtgc gggctgccag agccagatac gaggcctcta cagagcccgt gcctctgtac 2340

gtgccagagc tgggagatcc cgccagacag taccgggccc tgatcaacct gatctactgc 2400

cccgaccggg accccattgc ctggctgcag aatcctaagc tgaccggcgt gaacagcgcc 2460

ctgaaccagt tctaccagaa gctgctgcct cctggcagag ccggaacagc cgtgacagga 2520

tctgtggcct ctccagtgcc tcacgtggga gaagccatgg ccacaggcga agctctgtgg 2580

gccctgcctc atgctgctgc agctgtggct atgagcagaa gatacgaccg cgcccagaag 2640

cacttcatcc tgcagagcct gcggagagcc ttcgccagca tggcctatcc tgaggccacc 2700

ggatctagcc cagccgccag aatcagcaga ggccacccta gccctaccac ccctgccaca 2760

cagacccctg atcctcagcc tagcgctgcc gccagatctc tgagcgtgtg cccccctgac 2820

gacagactgc ggacccctag aaagcggaag tcccagcccg tggaatcccg gtccctgctg 2880

gacaagatca gagaaacccc cgtggccgac gccagggtgg ccgatgatca tgtggtgtcc 2940

aaggccaagc ggcgggtgtc cgagcctgtg acaatcacaa gcggccctgt ggtggacccc 3000

cctgccgtga tcacaatgcc actggacgga cctgccccca acggcggctt cagaagaatt 3060

cctagaggcg ccctgcacac ccccgtgcct agcgatcagg ccagaaaagc ctactgcacc 3120

cccgagacaa tcgccagact ggtggacgac cctctgttcc caacagcctg gcggcctgcc 3180

ctgtcttttg atcctggcgc tctggccgaa atcgccgcta gaaggccagg cggaggcgac 3240

agaagatttg gccctcctag cggcgtggaa gccctgaggc gaagatgcgc ctggatgaga 3300

cagatccccg accccgaaga tgtgcggctg ctgatcatct acgaccccct gcctggcgag 3360

gacatcaacg gcccactgga aagcaccctg gccaccgacc ctggaccatc ctggtcacct 3420

tctagaggcg gactgtctgt ggtgctggcc gccctgagca acagactgtg tctgccttct 3480

acacacgcct gggccggcaa ttggacaggc cctcctgatg tgtccgccct gaatgccaga 3540

ggcgtcctgc tgctgtctac ccgggatctc gctttcgctg gcgccgtgga atacctgggc 3600

tctagactgg ctagcgctcg gcggagactg ctggtcctgg atgctgtggc cctggaacgg 3660

tggcctagag atggcccagc cctgagccag taccatgtgt atgtgcgcgc tcccgccagg 3720

cctgatgcac aggcagtcgt cagatggccc gattctgccg tgaccgaagg actggccaga 3780

gccgtgtttg ccagctccag aacattcggc ccagccagct tcgcccggat cgagacagcc 3840

tttgccaacc tgtaccccgg cgagcagccc ctgtgtctgt gtagaggcgg caacgtggcc 3900

tacaccgtgt gtaccagagc cggccctaag accagggtgc cactgagccc tcgggaatac 3960

cggcagtacg tgctgcccgg cttcgacggc tgtaaagacc tggccagaca gagcaggggc 4020

ctgggactgg gagccgctga ttttgtggat gaggccgccc acagccacag agccgccaat 4080

agatggggac tgggcgctgc tctgaggccc gtgtttctgc cagagggtag acgaccaggc 4140

gccgctggac ctgaagcagg ggatgtgcct acctgggcca gagtgttttg tcggcacgct 4200

ctgctggaac ccgatccagc cgctgaacct ctggtgctgc cacctgtggc tggaagatcc 4260

gtggccctgt acgcctctgc cgacgaagcc agaaatgccc tgccccccat ccctagagtg 4320

atgtggcctc ctggattcgg cgctgccgag acagtgctgg aaggcagcga cggcaccaga 4380

tttgtgtttg gccaccacgg cggaagcgag cggcctgctg aaacacaggc tggccggcag 4440

agaagaaccg ccgacgatag ggaacacgcc ctcgagcccg atgactggga agtgggctgt 4500

gaagatgcct gggacagcga agagggtggc ggagatgatg gcgacgcccc tggcagctcc 4560

ttcggagtgt ctatcgtgtc tgtggcccct ggcgtgctgc gggacagaag agtgggactg 4620

aggccagccg tgaaggtgga actgctgagc agctcctcca gctccgagga tgaggatgac 4680

gtgtggggcg gaaggggcgg cagaagccct ccacagagta gaggatgatg agcggccgct 4740

cgagtctaga gggcccgttt aaacccgctg atcagcctcg actgtgcctt ctagttgcca 4800

gccatctgtt gtttgcccct cccccgtgcc ttccttgacc ctggaaggtg ccactcccac 4860

tgtcctttcc taataaaatg aggaaattgc atcgcattgt ctgagtaggt gtcattctat 4920

tctggggggt ggggtggggc aggacagcaa gggggaggat tgggaagaca atagcaggca 4980

tgctggggat gcggtgggct ctatggcttc tactgggcgg ttttatggac agcaagcgaa 5040

ccggaattgc cagctggggc gccctctggt aaggttggga agccctgcaa agtaaactgg 5100

atggctttct tgccgccaag gatctgatgg cgcaggggat caagctctga tcaagagaca 5160

ggatgaggat cgtttcgcat gattgaacaa gatggattgc acgcaggttc tccggccgct 5220

tgggtggaga ggctattcgg ctatgactgg gcacaacaga caatcggctg ctctgatgcc 5280

gccgtgttcc ggctgtcagc gcaggggcgc ccggttcttt ttgtcaagac cgacctgtcc 5340

ggtgccctga atgaactgca agacgaggca gcgcggctat cgtggctggc cacgacgggc 5400

gttccttgcg cagctgtgct cgacgttgtc actgaagcgg gaagggactg gctgctattg 5460

ggcgaagtgc cggggcagga tctcctgtca tctcaccttg ctcctgccga gaaagtatcc 5520

atcatggctg atgcaatgcg gcggctgcat acgcttgatc cggctacctg cccattcgac 5580

caccaagcga aacatcgcat cgagcgagca cgtactcgga tggaagccgg tcttgtcgat 5640

caggatgatc tggacgaaga gcatcagggg ctcgcgccag ccgaactgtt cgccaggctc 5700

aaggcgagca tgcccgacgg cgaggatctc gtcgtgaccc atggcgatgc ctgcttgccg 5760

aatatcatgg tggaaaatgg ccgcttttct ggattcatcg actgtggccg gctgggtgtg 5820

gcggaccgct atcaggacat agcgttggct acccgtgata ttgctgaaga gcttggcggc 5880

gaatgggctg accgcttcct cgtgctttac ggtatcgccg ctcccgattc gcagcgcatc 5940

gccttctatc gccttcttga cgagttcttc tgaattatta acgcttacaa tttcctgatg 6000

cggtattttc tccttacgca tctgtgcggt atttcacacc gcatcaggtg gcacttttcg 6060

gggaaatgtg cgcggaaccc ctatttgttt atttttctaa atacattcaa atatgtatcc 6120

gctcatgaga caataaccct gataaatgct tcaataatag cacgtgctaa aacttcattt 6180

ttaatttaaa aggatctagg tgaagatcct ttttgataat ctcatgacca aaatccctta 6240

acgtgagttt tcgttccact gagcgtcaga ccccgtagaa aagatcaaag gatcttcttg 6300

agatcctttt tttctgcgcg taatctgctg cttgcaaaca aaaaaaccac cgctaccagc 6360

ggtggtttgt ttgccggatc aagagctacc aactcttttt ccgaaggtaa ctggcttcag 6420

cagagcgcag ataccaaata ctgttcttct agtgtagccg tagttaggcc accacttcaa 6480

gaactctgta gcaccgccta catacctcgc tctgctaatc ctgttaccag tggctgctgc 6540

cagtggcgat aagtcgtgtc ttaccgggtt ggactcaaga cgatagttac cggataaggc 6600

gcagcggtcg ggctgaacgg ggggttcgtg cacacagccc agcttggagc gaacgaccta 6660

caccgaactg agatacctac agcgtgagct atgagaaagc gccacgcttc ccgaagggag 6720

aaaggcggac aggtatccgg taagcggcag ggtcggaaca ggagagcgca cgagggagct 6780

tccaggggga aacgcctggt atctttatag tcctgtcggg tttcgccacc tctgacttga 6840

gcgtcgattt ttgtgatgct cgtcaggggg gcggagccta tggaaaaacg ccagcaacgc 6900

ggccttttta cggttcctgg ccttttgctg gccttttgct cacatgttct t 6951

<210> 5
<211> 3855
<212> DNA
<213> Artificial Sequence

<220>
<223> pGX-5102

<400> 5
gctgcttcgc gatgtacggg ccagatatac gcgttgacat tgattattga ctagttatta 60

atagtaatca attacggggt cattagttca tagcccatat atggagttcc gcgttacata 120

acttacggta aatggcccgc ctggctgacc gcccaacgac ccccgcccat tgacgtcaat 180

aatgacgtat gttcccatag taacgccaat agggactttc cattgacgtc aatgggtgga 240

gtatttacgg taaactgccc acttggcagt acatcaagtg tatcatatgc caagtacgcc 300

ccctattgac gtcaatgacg gtaaatggcc cgcctggcat tatgcccagt acatgacctt 360

atgggacttt cctacttggc agtacatcta cgtattagtc atcgctatta ccatggtgat 420

gcggttttgg cagtacatca atgggcgtgg atagcggttt gactcacggg gatttccaag 480

tctccacccc attgacgtca atgggagttt gttttggcac caaaatcaac gggactttcc 540

aaaatgtcgt aacaactccg ccccattgac gcaaatgggc ggtaggcgtg tacggtggga 600

ggtctatata agcagagctc tctggctaac tagagaaccc actgcttact ggcttatcga 660

aattaatacg actcactata gggagaccca agctggctag cgtttaaact taagcttggt 720

accgagctcg gatccgccac catggactgg acctggattc tgttcctggt ggccgctgcc 780

acccgggtgc acagcatgtt ctgcaccagc cccgccacca gaggcgacag cagcgagtct 840

aagcctggcg ccagcgtgga cgtgaacggc aagatggaat acggcagcgc ccctggcccc 900

ctgaacggca gagatacaag cagaggccct ggcgccttct gcacccccgg atgggagatc 960

caccccgcca gactggtgga agatatcaac cgggtgttcc tgtgtatcgc ccagagcagc 1020

ggcagagtga cccgggacag cagacggctg cggcggatct gcctggactt ctacctgatg 1080

ggccggaccc ggcagaggcc taccctggcc tgttgggagg aactgctgca gctgcagccc 1140

acccagaccc agtgcctgcg ggccaccctg atggaagtgt cccacagacc ccccagaggc 1200

gaggacggct tcatcgaggc ccccaacgtg cccctgcacc gcagcgctct ggaatgcgac 1260

gtgtccgacg acggcggcga ggacgacagc gacgatgatg gcagcacccc cagcgacgtg 1320

atcgagttca gagacagcga cgccgagagc agcgacggcg aggacttcat cgtggaagag 1380

gaaagcgagg aatccaccga cagctgcgag cccgatggcg tgcccggcga ctgctataga 1440

gatggcgacg gctgcaacac ccccagcccc aagaggcccc agcgggccat cgaaagatac 1500

gctggcgccg agacagccga gtacaccgcc gccaaagccc tgacagccct gggcgaaggc 1560

ggcgtggact ggaagagaag aaggcacgag gcccctcggc ggcacgacat tcctccacct 1620

catggcgtgt agtgagcggc cgctcgagtc tagagggccc gtttaaaccc gctgatcagc 1680

ctcgactgtg ccttctagtt gccagccatc tgttgtttgc ccctcccccg tgccttcctt 1740

gaccctggaa ggtgccactc ccactgtcct ttcctaataa aatgaggaaa ttgcatcgca 1800

ttgtctgagt aggtgtcatt ctattctggg gggtggggtg gggcaggaca gcaaggggga 1860

ggattgggaa gacaatagca ggcatgctgg ggatgcggtg ggctctatgg cttctactgg 1920

gcggttttat ggacagcaag cgaaccggaa ttgccagctg gggcgccctc tggtaaggtt 1980

gggaagccct gcaaagtaaa ctggatggct ttcttgccgc caaggatctg atggcgcagg 2040

ggatcaagct ctgatcaaga gacaggatga ggatcgtttc gcatgattga acaagatgga 2100

ttgcacgcag gttctccggc cgcttgggtg gagaggctat tcggctatga ctgggcacaa 2160

cagacaatcg gctgctctga tgccgccgtg ttccggctgt cagcgcaggg gcgcccggtt 2220

ctttttgtca agaccgacct gtccggtgcc ctgaatgaac tgcaagacga ggcagcgcgg 2280

ctatcgtggc tggccacgac gggcgttcct tgcgcagctg tgctcgacgt tgtcactgaa 2340

gcgggaaggg actggctgct attgggcgaa gtgccggggc aggatctcct gtcatctcac 2400

cttgctcctg ccgagaaagt atccatcatg gctgatgcaa tgcggcggct gcatacgctt 2460

gatccggcta cctgcccatt cgaccaccaa gcgaaacatc gcatcgagcg agcacgtact 2520

cggatggaag ccggtcttgt cgatcaggat gatctggacg aagagcatca ggggctcgcg 2580

ccagccgaac tgttcgccag gctcaaggcg agcatgcccg acggcgagga tctcgtcgtg 2640

acccatggcg atgcctgctt gccgaatatc atggtggaaa atggccgctt ttctggattc 2700

atcgactgtg gccggctggg tgtggcggac cgctatcagg acatagcgtt ggctacccgt 2760

gatattgctg aagagcttgg cggcgaatgg gctgaccgct tcctcgtgct ttacggtatc 2820

gccgctcccg attcgcagcg catcgccttc tatcgccttc ttgacgagtt cttctgaatt 2880

attaacgctt acaatttcct gatgcggtat tttctcctta cgcatctgtg cggtatttca 2940

caccgcatca ggtggcactt ttcggggaaa tgtgcgcgga acccctattt gtttattttt 3000

ctaaatacat tcaaatatgt atccgctcat gagacaataa ccctgataaa tgcttcaata 3060

atagcacgtg ctaaaacttc atttttaatt taaaaggatc taggtgaaga tcctttttga 3120

taatctcatg accaaaatcc cttaacgtga gttttcgttc cactgagcgt cagaccccgt 3180

agaaaagatc aaaggatctt cttgagatcc tttttttctg cgcgtaatct gctgcttgca 3240

aacaaaaaaa ccaccgctac cagcggtggt ttgtttgccg gatcaagagc taccaactct 3300

ttttccgaag gtaactggct tcagcagagc gcagatacca aatactgttc ttctagtgta 3360

gccgtagtta ggccaccact tcaagaactc tgtagcaccg cctacatacc tcgctctgct 3420

aatcctgtta ccagtggctg ctgccagtgg cgataagtcg tgtcttaccg ggttggactc 3480

aagacgatag ttaccggata aggcgcagcg gtcgggctga acggggggtt cgtgcacaca 3540

gcccagcttg gagcgaacga cctacaccga actgagatac ctacagcgtg agctatgaga 3600

aagcgccacg cttcccgaag ggagaaaggc ggacaggtat ccggtaagcg gcagggtcgg 3660

aacaggagag cgcacgaggg agcttccagg gggaaacgcc tggtatcttt atagtcctgt 3720

cgggtttcgc cacctctgac ttgagcgtcg atttttgtga tgctcgtcag gggggcggag 3780

cctatggaaa aacgccagca acgcggcctt tttacggttc ctggcctttt gctggccttt 3840

tgctcacatg ttctt 3855

<210> 6
<211> 4890
<212> DNA
<213> Artificial Sequence

<220>
<223> pGX-5103

<400> 6
gctgcttcgc gatgtacggg ccagatatac gcgttgacat tgattattga ctagttatta 60

atagtaatca attacggggt cattagttca tagcccatat atggagttcc gcgttacata 120

acttacggta aatggcccgc ctggctgacc gcccaacgac ccccgcccat tgacgtcaat 180

aatgacgtat gttcccatag taacgccaat agggactttc cattgacgtc aatgggtgga 240

gtatttacgg taaactgccc acttggcagt acatcaagtg tatcatatgc caagtacgcc 300

ccctattgac gtcaatgacg gtaaatggcc cgcctggcat tatgcccagt acatgacctt 360

atgggacttt cctacttggc agtacatcta cgtattagtc atcgctatta ccatggtgat 420

gcggttttgg cagtacatca atgggcgtgg atagcggttt gactcacggg gatttccaag 480

tctccacccc attgacgtca atgggagttt gttttggcac caaaatcaac gggactttcc 540

aaaatgtcgt aacaactccg ccccattgac gcaaatgggc ggtaggcgtg tacggtggga 600

ggtctatata agcagagctc tctggctaac tagagaaccc actgcttact ggcttatcga 660

aattaatacg actcactata gggagaccca agctggctag cgtttaaact taagcttggt 720

accgagctcg gatccgccac catggactgg acctggattc tgttcctggt ggccgctgcc 780

acccgggtgc acagcatggg caccgtgaac aagcccgtcg tgggagtgct gatgggcttc 840

ggcatcatca ccggcaccct gcggatcacc aaccccgtgc gggccagcgt gctgagatac 900

gacgacttcc acatcgacga ggacaagctg gacaccaaca gcgtgtacga gccctactac 960

cacagcgacc acgccgagag cagctgggtg aaccggggcg agagcagccg gaaggcctac 1020

gaccacaaca gcccctacat ctggccccgg aacgactacg acggcttcct ggaaaacgcc 1080

cacgagcacc acggcgtgta caaccagggc agaggcatcg acagcggcga gcggctgatg 1140

cagcctaccc agatgagcgc ccaggaagat ctgggcgacg acaccggcat ccacgtgatc 1200

cccaccctga acggcgacga ccggcacaag atcgtgaacg tggaccagcg gcagtacggc 1260

gacgtgttca agggcgacct gaaccccaag ccccagggcc agagactgat cgaggtgtcc 1320

gtggaagaga accacccctt caccctgaga gcccccatcc agcggatcta cggcgtgcgg 1380

tataccgaga cttggagctt cctgcccagc ctgacctgca ccggggatgc cgcccctgcc 1440

atccagcaca tctgcctgaa gcacaccacc tgtttccagg acgtggtggt ggacgtggac 1500

tgcgccgaga acaccaaaga ggaccagctg gccgagatca gctaccggtt ccagggcaag 1560

aaagaggccg accagccctg gatcgtggtg aacaccagca ccctgttcga cgagctggaa 1620

ctggaccccc ccgagatcga gcccggcgtg ctgaaggtgc tgcggaccga gaagcagtac 1680

ctgggcgtgt acatctggaa catgcggggc agcgacggca ccagcaccta cgccaccttt 1740

ctggtgacat ggaagggcga cgagaaaacc cggaacccca cccctgccgt gacccctcag 1800

cctagaggcg ccgagtttca catgtggaac taccactccc acgtgttcag cgtgggcgac 1860

accttcagcc tggccatgca tctgcagtac aagatccacg aggccccctt cgacctgctg 1920

ctggaatggc tgtacgtgcc catcgaccct acctgccagc ccatgcggct gtacagcacc 1980

tgtctgtacc accccaacgc cccccagtgc ctgagccaca tgaacagcgg ctgcaccttc 2040

accagccccc acctggctca gagggtggcc agcaccgtgt accagaactg cgagcacgcc 2100

gacaactaca ccgcctactg cctgggcatc agccacatgg aacccagctt cggcctgatc 2160

ctgcacgacg gcggcaccac cctgaagttc gtggacaccc cagagagcct gagcggcctg 2220

tacgtgttcg tggtgtactt caacggccac gtggaagccg tggcctacac cgtggtgtcc 2280

accgtggacc acttcgtgaa cgccatcgag gaacggggct tcccacccac agccggacag 2340

cctccagcca ccaccaagcc caaagaaatc acccccgtga accccggcac cagccctctg 2400

ctgagatatg ccgcctggac aggcggactg gccgctgtgg tgctgctgtg cctggtgatc 2460

ttcctgatct gcaccgccaa gcggatgaga gtgaaggcct accgggtgga caagtccccc 2520

tacaaccaga gcatgtacta cgccggcctg cccgtggacg atttcgagga cagcgagagc 2580

accgacaccg aggaagagtt cggcaacgcc atcggcggca gccacggcgg ctctagctac 2640

accgtgtaca tcgacaagac ccgctgatga gcggccgctc gagtctagag ggcccgttta 2700

aacccgctga tcagcctcga ctgtgccttc tagttgccag ccatctgttg tttgcccctc 2760

ccccgtgcct tccttgaccc tggaaggtgc cactcccact gtcctttcct aataaaatga 2820

ggaaattgca tcgcattgtc tgagtaggtg tcattctatt ctggggggtg gggtggggca 2880

ggacagcaag ggggaggatt gggaagacaa tagcaggcat gctggggatg cggtgggctc 2940

tatggcttct actgggcggt tttatggaca gcaagcgaac cggaattgcc agctggggcg 3000

ccctctggta aggttgggaa gccctgcaaa gtaaactgga tggctttctt gccgccaagg 3060

atctgatggc gcaggggatc aagctctgat caagagacag gatgaggatc gtttcgcatg 3120

attgaacaag atggattgca cgcaggttct ccggccgctt gggtggagag gctattcggc 3180

tatgactggg cacaacagac aatcggctgc tctgatgccg ccgtgttccg gctgtcagcg 3240

caggggcgcc cggttctttt tgtcaagacc gacctgtccg gtgccctgaa tgaactgcaa 3300

gacgaggcag cgcggctatc gtggctggcc acgacgggcg ttccttgcgc agctgtgctc 3360

gacgttgtca ctgaagcggg aagggactgg ctgctattgg gcgaagtgcc ggggcaggat 3420

ctcctgtcat ctcaccttgc tcctgccgag aaagtatcca tcatggctga tgcaatgcgg 3480

cggctgcata cgcttgatcc ggctacctgc ccattcgacc accaagcgaa acatcgcatc 3540

gagcgagcac gtactcggat ggaagccggt cttgtcgatc aggatgatct ggacgaagag 3600

catcaggggc tcgcgccagc cgaactgttc gccaggctca aggcgagcat gcccgacggc 3660

gaggatctcg tcgtgaccca tggcgatgcc tgcttgccga atatcatggt ggaaaatggc 3720

cgcttttctg gattcatcga ctgtggccgg ctgggtgtgg cggaccgcta tcaggacata 3780

gcgttggcta cccgtgatat tgctgaagag cttggcggcg aatgggctga ccgcttcctc 3840

gtgctttacg gtatcgccgc tcccgattcg cagcgcatcg ccttctatcg ccttcttgac 3900

gagttcttct gaattattaa cgcttacaat ttcctgatgc ggtattttct ccttacgcat 3960

ctgtgcggta tttcacaccg catcaggtgg cacttttcgg ggaaatgtgc gcggaacccc 4020

tatttgttta tttttctaaa tacattcaaa tatgtatccg ctcatgagac aataaccctg 4080

ataaatgctt caataatagc acgtgctaaa acttcatttt taatttaaaa ggatctaggt 4140

gaagatcctt tttgataatc tcatgaccaa aatcccttaa cgtgagtttt cgttccactg 4200

agcgtcagac cccgtagaaa agatcaaagg atcttcttga gatccttttt ttctgcgcgt 4260

aatctgctgc ttgcaaacaa aaaaaccacc gctaccagcg gtggtttgtt tgccggatca 4320

agagctacca actctttttc cgaaggtaac tggcttcagc agagcgcaga taccaaatac 4380

tgttcttcta gtgtagccgt agttaggcca ccacttcaag aactctgtag caccgcctac 4440

atacctcgct ctgctaatcc tgttaccagt ggctgctgcc agtggcgata agtcgtgtct 4500

taccgggttg gactcaagac gatagttacc ggataaggcg cagcggtcgg gctgaacggg 4560

gggttcgtgc acacagccca gcttggagcg aacgacctac accgaactga gatacctaca 4620

gcgtgagcta tgagaaagcg ccacgcttcc cgaagggaga aaggcggaca ggtatccggt 4680

aagcggcagg gtcggaacag gagagcgcac gagggagctt ccagggggaa acgcctggta 4740

tctttatagt cctgtcgggt ttcgccacct ctgacttgag cgtcgatttt tgtgatgctc 4800

gtcagggggg cggagcctat ggaaaaacgc cagcaacgcg gcctttttac ggttcctggc 4860

cttttgctgg ccttttgctc acatgttctt 4890

## Description

### [Technical Field]

The present invention relates to technology in the vaccination field, and more particularly, to a vaccine for preventing and treating herpes zoster which has excellent productivity, stability and immune reactivity against T cells and is applicable to various conditions of diseases.

### [Background Art]

In general, herpes zoster is a skin rash disease that develops as a varicella-zoster virus (VZV) lies dormant in the neuronal ganglion until the immune system becomes weak, and then the reactivation of VZV is induced. When the herpes zoster develops, bullous lesions are formed, and patients have the aftereffects of postherpetic neuralgia even when the herpes zoster is restored. In this case, it is difficult to completely cure the postherpetic neuralgia once the postherpetic neuralgia develops, and thus the patients experience extreme pain. The reactivation of VZV and the onset of herpes zoster are associated with the weakening of cellular immune response in T cells. In particular, the herpes zoster is a disease that frequently develops in the aged or persons whose receive an immunosuppressive therapy.

When a patient develops herpes zoster, the patient will receive a treatment with an antiviral drug. However, this purpose of treatment is basically to prevent a rash from spreading to other sites by administering the antiviral drug within 72 hours after the patient begins to get a rash, and to reduce or alleviate the complications after the onset of herpes zoster. As the aged and immunosuppressed patients increase, the incidence rate of herpes zoster in Korea has increased rapidly, but there are no fundamental medications to treat the herpes zoster. Therefore, the herpes zoster is considered to be a disease that emphasizes the great importance of prophylactic vaccines.

Prophylactic vaccines against herpes zoster currently on the market have a medicinal effect approved in clinical trials, but the rate of decline in the onset of herpes zoster in response to the administration of the prophylactic vaccines reaches up to 50%. Therefore, there is a need for development of a novel vaccine against herpes zoster which has an excellent medicinal effect. Also, since the prophylactic vaccines against herpes zoster currently on the market are live attenuated vaccines, the prophylactic vaccines are in a paradoxical situation in which they cannot be rather used for immunosuppressed patients having a high incidence rate of herpes zoster. Accordingly, there is an urgent demand for development of a novel vaccine against herpes zoster which can be safely administered to the immunosuppressed patients.

### [Disclosure]

### [Technical Problem]

Therefore, the present invention is designed to solve the problems of the prior art, and it is an object of the present invention to provide a DNA vaccine composition for preventing and treating herpes zoster, which is capable of being applied to patients having various types of diseases and has remarkably improved stability.

It is another object of the present invention to provide a method for activating T cells against a VZV antigen by effectively administering the DNA vaccine composition for preventing and treating herpes zoster into the body.

### [Technical Solution]

According to an aspect of the present invention, there is provided a plasmid DNA for preventing and treating herpes zoster, which contains an insertion site of a varicella-zoster virus (VZV)-derived gene encoding a VZV protein, wherein the plasmid DNA is directly administered into the body by an electroporation method to induce immune activity of T cells against a VZV antigen.

An electroporation system (CELLECTRA commercially available from Inovio Pharmaceuticals Inc.) was used as an electroporation system for the electroporation.

The gene may include one selected from the group consisting a first gene encoding an IE-62 protein (SEQ ID NO: 1), a second gene encoding an IE-63 protein (SEQ ID NO: 2), and a third gene encoding a gE protein (SEQ ID NO: 3).

Specifically, the plasmid may include one selected from the group consisting a plasmid DNA set forth in SEQ ID NO: 4, a plasmid DNA set forth in SEQ ID NO: 5, and 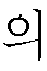 plasmid DNA set forth in SEQ ID NO: 6.

According to another aspect of the present invention, there is provided a DNA vaccine composition for preventing and treating herpes zoster, which includes at least one plasmid containing an insertion site of a varicella-zoster virus (VZV)-derived gene encoding a VZV protein, and other pharmaceutically acceptable ingredients. The other ingredients may, for example, include water such as saline, etc.

The DNA vaccine composition may include a plurality of plasmids, which contain different insertion sites of heterologous genes, as the plasmid constituting the DNA vaccine. For example, a plurality of plasmids containing different genetic loci encoding the VZV protein may be used as the plasmid.

Three plasmids such as a first plasmid containing an insertion site of a first gene encoding an IE-62 protein (SEQ ID NO: 1), a second plasmid containing an insertion site of a second gene encoding an IE-63 protein (SEQ ID NO: 2), and a third plasmid containing an insertion site of a third gene encoding a gE protein (SEQ ID NO: 3) may be used as the plasmid included in the DNA vaccine composition. A content of each of the plasmids in the composition may be variously adjusted in consideration of the type of a disease, the age of a subject to which the plasmid is administered, etc.

According to still another aspect of the present invention, there is provided a method for activating T cells against a varicella-zoster virus (VZV) antigen, which includes preparing a plasmid containing a VZV-derived gene encoding a VZV protein, and administering the plasmid into the body using an electroporation method. In this case, the plasmid may be mass-produced using a mass production system.

### [Advantageous Effects]

The DNA vaccine for preventing and treating herpes zoster according to the present invention does not cause safety issues caused by live attenuated vaccines since the DNA vaccine is a vaccine delivered through direct administration of a plasmid. Also, the plasmid can be easily mass-produced, and stability of the vaccine during a distribution process can be remarkably improved. Since the DNA vaccine is administered into the body using an electroporation method, the DNA vaccine can exhibit remarkable *in vivo* delivery efficiency, compared to conventional injectable drugs. Meanwhile, the DNA vaccine for preventing and treating herpes zoster can be used for a therapeutic purpose as well as a prophylactic purpose, and may be properly used for patients having various diseases, and patients of different ages. The DNA vaccine for preventing and treating herpes zoster has an advantage in that personalized vaccines can be designed through various combinations of a plurality of plasmids containing different insertion sites of plasmid genes.

### [Description of Drawings]

FIG. 1 is a diagram showing a vaccination schedule according to one exemplary embodiment of the present invention.
FIG. 2 shows graphs illustrating results of immunogenicity for experimental groups according to one exemplary embodiment of the present invention.
FIGS. 3 and 4 are graphs illustrating results of comparing immune responses of overlapping peptides of proteins in respective experimental groups.
FIGS. 5 to 7 are electrophoretic image of products expressed by respective plasmid genes.

### [Best Mode]

Hereinafter, a DNA vaccine for preventing and treating herpes zoster will be described in detail.

The DNA vaccine for preventing and treating herpes zoster according to the present invention is not a virus-based live vaccine but a DNA-based vaccine in which DNA itself is administered into the body. As a result, the DNA itself in the form of a plasmid, which is administered into the body to induce immune activity of T cells against a VZV antigen, serves as the vaccine.

The DNA vaccine encodes a VZV-derived antigen protein, and ultimately plays a role in forming the antigen protein in the body. The VZV-derived protein is preferably chosen in consideration of desired characteristics of the DNA vaccine. In this case, the protein may be chosen alone or in combination with plural types thereof.

According to one exemplary embodiment of the present invention, the protein may include proteins IE-62 (SEQ ID NO: 1) and IE-63 (SEQ ID NO: 2) and a glucoprotein gE (SEQ ID NO: 3), all of which may induce a cellular immune response, and the plasmid is designed so that a gene encoding such a protein can be inserted into the plasmid.

The plasmid DNA is administered so that the plasmid DNA is mixed with a liquid in the body. For this purpose, the plasmid DNA of the present invention may be designed to be introduced into the body with high efficiency using an electroporation method. Only when such an electroporation method is used, *in vivo* delivery efficiency may be maximized, thereby maximizing therapeutic efficacy. A site of the body to which the plasmid DNA is administered using the electroporation method includes a region of muscle, etc., but the present invention is not particularly limited thereto. For example, the site of the body may be determined in consideration of various factors such as the age of a subject to which the plasmid DNA is administered, the type of a disease, concurrent diseases, etc. An electroporation system (CELLECTRA commercially available from Inovio Pharmaceuticals Inc.) may be used as electroporation equipment used to realize the electroporation method.

The genes, for example, a first gene, a second gene and a third gene which may encode and express the IE-62, IE-63 and gE proteins, respectively, are incorporated into the plasmid using a conventional method. However, to maximize the medicinal effect and administration efficiency, plasmids set forth, respectively, in SEQ ID NO: 4, SEQ ID NO: 5 and SEQ ID NO: 6 may be designed in this example to maximize the efficiency of the DNA vaccine. The respective plasmids may be used alone, but are preferably included in the DNA vaccine in a combination of two or more different plasmids. For example, the DNA vaccine containing all the three plasmids may be contemplated in one exemplary embodiment of the present invention. Meanwhile, different types of plasmids which are not specified in this example may also be newly introduced.

The plasmid may be administered into the body by means of an injectable drug, etc. In this case, the plasmid should be prepared with a high concentration and high purity to improve delivery efficiency and immune response efficiency. Also, the ease in mass production should be ensured in a commercial aspect. The plasmid according to one exemplary embodiment of the present invention has a foundation to be mass-produced with such a high concentration and high purity.

The DNA vaccine for preventing and treating herpes zoster according to one exemplary embodiment of the present invention is a vaccine for liquids, that is, injectable drugs, which includes at least one plasmid DNA as described above. Thus, the DNA vaccine includes the plasmid DNA and a liquid ingredient. Here, the liquid ingredient may be pure water. In addition to the water, the DNA vaccine composition according to one exemplary embodiment of the present invention may further include pharmaceutically acceptable functional additives. In this case, such additives may be introduced at a level known in the related art.

The DNA vaccine composition may, for example, include all the three plasmids as described above.

Hereinafter, the efficiency of the plasmid DNA according to one exemplary embodiment of the present invention will be described based on the specific contents of experiments.

### [Experiment 1] Evaluation of immunogenicity

### Screening of VZV gene candidates usable in DNA vaccine

Genes of proteins IE-62 and IE-63 and a glucoprotein gE, all of which may induce a cellular immune response, were screened from 67 genes encoding proteins in the VZV genome. The type of the VZV genome was divided into a total of 5 clades, and there was no significant difference in amino acid sequences of the IE-62, IE-63 and gE between the clades. Plasmids were constructed using sequences of the aforementioned IE-62, IE-63 and gE genes. The amino acid sequences of the proteins are as set forth in SEQ ID NOs: 1 to 3, and the DNA sequences of the constructed plasmids are as set forth in SEQ ID NOs: 4 to 6. In this example, the DNA vaccine included the plasmid and a liquid ingredient (i.e., water), but did not include other ingredients.

### Experiment on mice

The IE-62, IE-63 and gE genes were prepared as candidate materials for the DNA vaccine, and administered into C57BL/6 6 mice. Thereafter, splenocytes were removed from the mice to evaluate immunogenicity of T cells against a VZV antigen. A total of 5 groups were used for this experiment, and each group consisted of five mice, as follows.
1) Neg: A group in which mice are vaccinated with a vector (n = 5)
2) IE62: A group in which mice are vaccinated with an IE-62 DNA vaccine (n = 5)
3) IE63: A group in which mice are vaccinated with an IE-63 DNA vaccine (n = 5)
4) gE: A group in which mice are vaccinated with a gE DNA vaccine (n = 5)
5) IE62+IE63+gE: A group in which mice are vaccinated with a mixture of IE-62, IE-63 and gE DNA vaccines

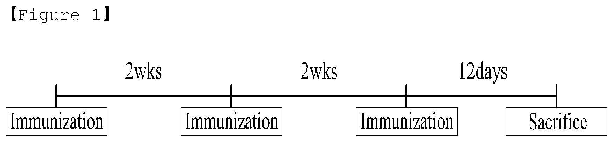

The vaccination was performed three times at an interval of 2 weeks using an electroporation system (CELLECTRA commercially available from Inovio Pharmaceuticals Inc.). In this case, an amount of the DNA was 30 µg/mice upon every vaccination, and the mixed group was vaccinated at 30 µg for each DNA (a total amount of 90 µg). After 12 days of the vaccination, splenocytes were removed to perform an immuoassay. A vaccination schedule is shown in FIG. 1. FIG. 1 is a diagram showing a vaccination schedule according to one exemplary embodiment of the present invention.

### Preparation of antigen for experiments

To systematically measure the entire immune response to proteins encoded by candidate genes, a series of overlapping peptides (OLPs) were designed and prepared. Each of the overlapping peptides was designed to consist of 15 amino acids with 10 amino acids overlapping with its adjacent peptides. Also, the overlapping peptides were designed to contain full-length amino acid sequences of the IE-62, IE-63 and gE proteins. As a result, 261 IE-62 overlapping peptides, 54 IE-63 overlapping peptides and 124 gE overlapping peptides were prepared. The 37 or 38 consecutive IE-62 overlapping peptides were mixed to prepare a total of 7 peptide mixtures, which were then used. The 27 consecutive IE-63 overlapping peptides were mixed to prepare a total of 2 peptide mixtures, which were then used. The 40 or 41 consecutive IE-63 overlapping peptides were mixed to prepare a total of 3 peptide mixtures, which were then used. 5% DMSO was used as the negative control, and a mixture of phorbol myristate acetate (PMA) and ionomycin was used as the positive control. Peptide mixtures prepared by mixing the overlapping peptides are listed in the following Table 1.

**[Table 1]**

| Proteins | Antigen names | SEQ ID NOs of amino acids | Number of mixed overlapping peptides |
|---|---|---|---|
| IE-62 | IE62-1 | 1-37 | 37 |
| | IE62-2 | 28-74 | 37 |
| | IE62-3 | 75-111 | 37 |
| | IE62-4 | 112-143 | 37 |
| | IE62-5 | 149-185 | 37 |
| | IE62-6 | 186-223 | 38 |
| | IE62-7 | 224-261 | 38 |
| IE-63 | IE63-1 | 1-27 | 27 |
| | IE63-2 | 28-54 | 27 |
| gE | gE -1 | 1-27 | 41 |
| | gE -2 | 28-54 | 40 |
| | gE -3 | 1-41 | 41 |

A lysate prepared after MRC-5 cells were infected with VZV was used as the antigen, and an MRC-5 cell lysate was used as a negative antigen corresponding to the lysate.

### IFN-γ ELISPOT assay protocol

Splenocytes were seeded at 1×10⁶ cells/well to perform an ELISPOT assay. When the splenocytes were stimulated with a mixture of overlapping peptides, the overlapping peptides were administered so that a concentration of each of the overlapping peptides reached 1 µg/ml per well, and, when the splenocytes were stimulated with a lysate antigen, the lysate antigen was administered so that a concentration of the lysate antigen reached 50 µg/ml per well. In this case, this experiment was repeated in triplicate. The assay results obtained through the stimulation with the overlapping peptide antigen was calculated, as follows: Mean of OLP pools-specific spot forming cells (SFCs) - Mean of 5% DMSO-specific SFCs. The assay results obtained through the stimulation with the lysate antigen was calculated, as follows: Mean of VZV-specific SFCs - Mean of MRC-5-specific SFCs. The maximum value of SFCs per well was calculated to be 500.

### Experimental results of immunogenicity

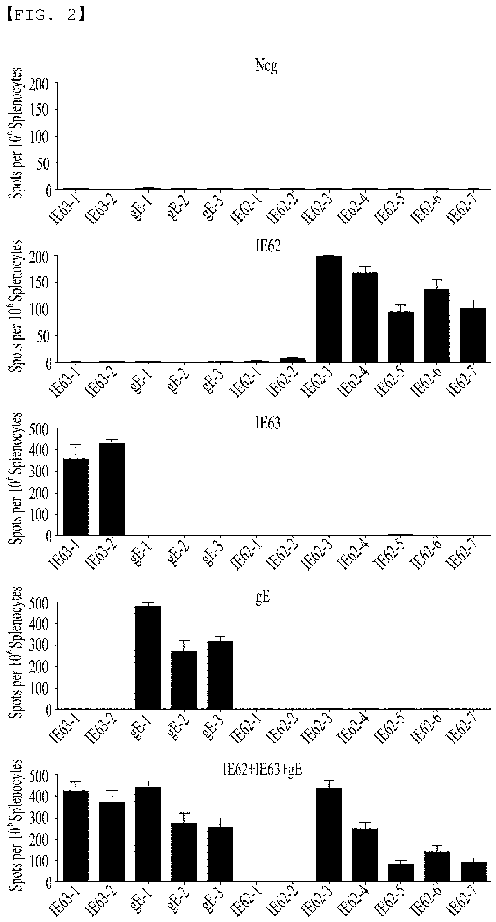

The results of antigen evaluation with respect to the respective vaccines showed that no immune response was induced with respect to the stimulus of overlapping peptides in the Neg-treated group used as the negative control. It was revealed that an immune response was induced with respect to the overlapping peptide of the corresponding protein in the IE-62-, IE-63- and gE-treated groups in which each of the IE-62, IE-63 and gE DNA vaccines were administered alone. It was revealed that an immune response was induced with respect to the overlapping peptides of the three proteins in the IE62+IE63+gE-treated group in which all the IE-62, IE-63 and gE DNA vaccines were administered. When a DNA vaccine of a candidate material was administered, an immune response was induced with respect to the overlapping peptides specific to the candidate material. FIG. 2 shows graphs illustrating results of immunogenicity for experimental groups according to one exemplary embodiment of the present invention.

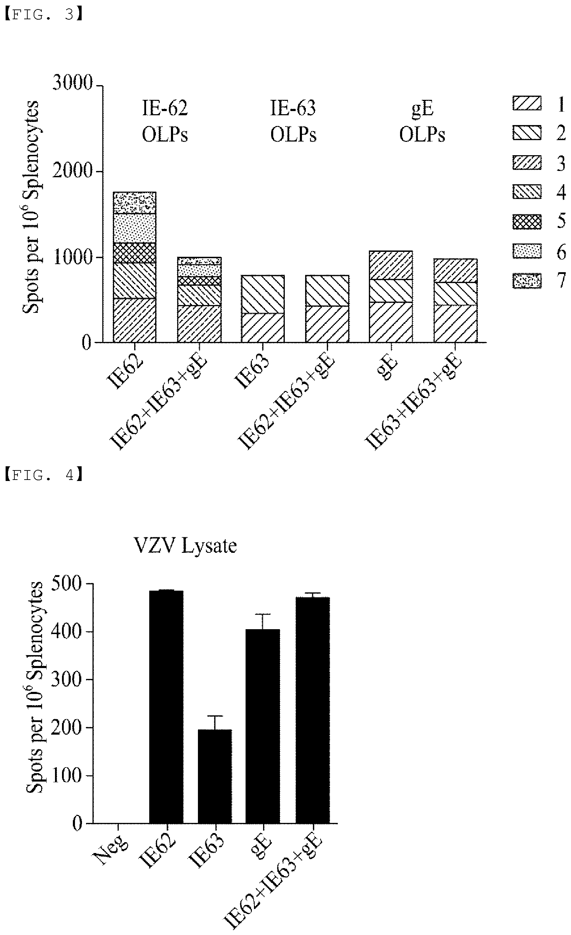

FIGS. 3 and 4 are graphs illustrating results of comparing immune responses of overlapping peptides of proteins in respective experimental groups. Referring to FIG. 3, the immune responses of the overlapping peptides of the corresponding proteins were compared in the groups in which a single DNA vaccine was administered and the group in which a mixture of three DNA vaccines was administered. As a result, it was revealed that, when the mixture of three DNA vaccines was administered, the immune response to IE-62 decreased by 43.3%, there was no difference in the immune response to IE-63, and the immune response to gE decreased by 9.6%, compared to when the single DNA vaccine was administered.

### Validity of selection of antigen

Referring to FIG. 4, the splenocytes were stimulated with the lysate antigen in each of the experimental groups. As a result, it was confirmed that no immune response was induced in the Neg-treated group, but a strong immune response was induced in the group in which the DNA vaccine of the candidate material was administered. From these result, it can be seen that the proteins such as IE-62, IE-63, gE and the like were expressed in the cells infected with VZV, and an immune response occurred in the cells infected with VZV when the immune response to IE-62, IE-63 and gE selected as the candidate materials was induced in this experiment.

### Experimental evaluation

In a laboratory animal model, the DNA vaccines prepared using the genes encoding the IE-62, IE-63 and gE proteins were evaluated and analyzed. As a result, it was revealed that the T cells exhibited sufficient immunogenicity when the DNA vaccines administered alone or in combination thereof. The immunogenicity of the T cells was analyzed through the stimulation of the VZV lysate antigen. As a result, it was judged that it was reasonable to select the IE-62, IE-63 and gE as the candidate materials for the vaccines.

### [Experiment 2] Evaluation of in vitro protein expression

### Criteria for expression confirmation

- Transfection: RD cells (1 × 10⁶ cells/T75 flask), 30 µg of pDNA, Lipofectamine 2000, cell harvesting after 24 hours of transfection
   * RD cells: human rhabdomyosarcoma
- Cell lysis: 2 × 10⁶ cells/90 µl of RIPA buffer with Protease Inhibitor Cocktail
- SDSPAGE: LDS sample buffer with Reducing agent (DTT), 30 µl loading (19.5 µl of lysate), 4 to 12% Bis-Tris gel, MES running buffer at 165 V for 35 minutes
- Protein transfer: NC membrane, Dry blotting at 20 V for 1 minute → at 23 V for 4 minutes → at 25 V for 2 minutes
- Protein detection: 1) Blocking for 1 hour, 2) Antibody reaction (1 : 100) for an hour and a half (HA-probe HRP (SantaCruz, Cat. No.: sc-805HRP), Goat anti-VZV IE62 (SantaCruz, Cat. No.: sc-17525), Donkeyanti-goat Ig GHRP (SantaCruz, Cat. No.: sc-2020), 3), and HRP chromogenic substrate (Invitrogen, Cat. No.: WP20004))

### Evaluation of Expression confirmation

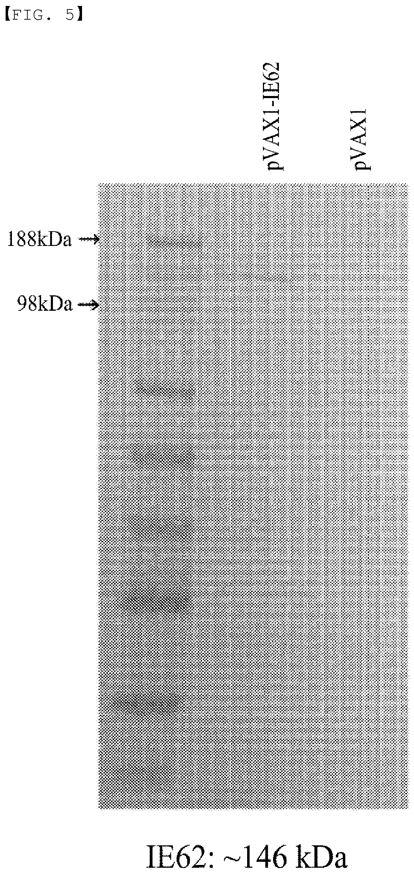

FIGS. 5 to 7 are electrophoretic image of products expressed by the respective plasmid genes.

Referring to FIG. 5, the plasmid encoding an IE-62 gene was introduced into RD cells derived from human rhabdomyosarcoma using Lipofectamine 2000. After 24 hours, the cells were lysed to extract proteins, and the proteins were then subjected to Western Blotting using an antibody for detecting an expressed antigen. An expressed protein of the plasmid encoding the IE-62 gene was detected using anti-VZV IE62 (SantaCruz, Cat. No.: SC-2020), and visualized using a HRP chromogenic substrate (Invitrogen, Cat. No.: WP20004). As a result, it was revealed that the product size of the insert gene expressed from the plasmid encoding the IE-62 gene was identical to the predicted molecular weight of the insert gene, indicating that the product of the gene introduced into the RD cells was the IE-62 antigen protein of VZV.

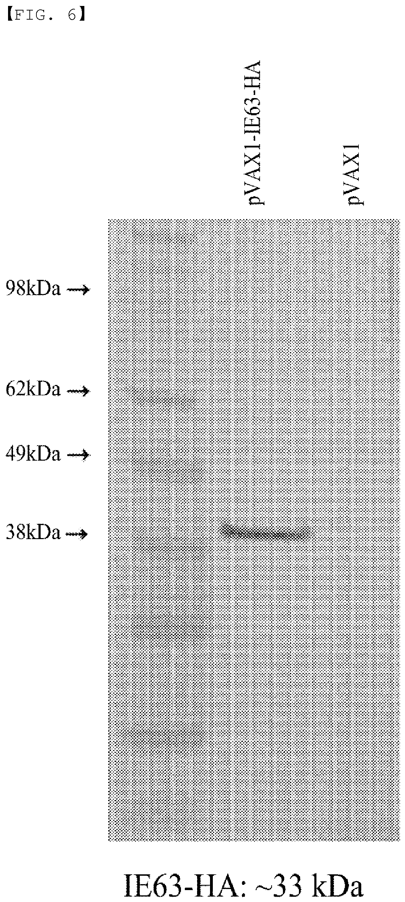

Referring to FIG. 6, the plasmid encoding an IE-63 gene was introduced into RD cells derived from human rhabdomyosarcoma using Lipofectamine 2000. After 24 hours, the cells were lysed to extract proteins, and the proteins were then subjected to Western Blotting using an antibody for detecting an expressed antigen. An expressed protein of the plasmid encoding the IE-63 gene was detected using HA-probe HRP (SantaCruz, Cat. No.: SC-805HRP), and visualized using a HRP chromogenic substrate (Invitrogen, Cat. No.: WP20004). As a result, it was revealed that the product size of the insert gene expressed from the plasmid encoding the IE-63 gene was identical to the predicted molecular weight of the insert gene, indicating that the product of the gene introduced into the RD cells was the IE-63 antigen protein of VZV.

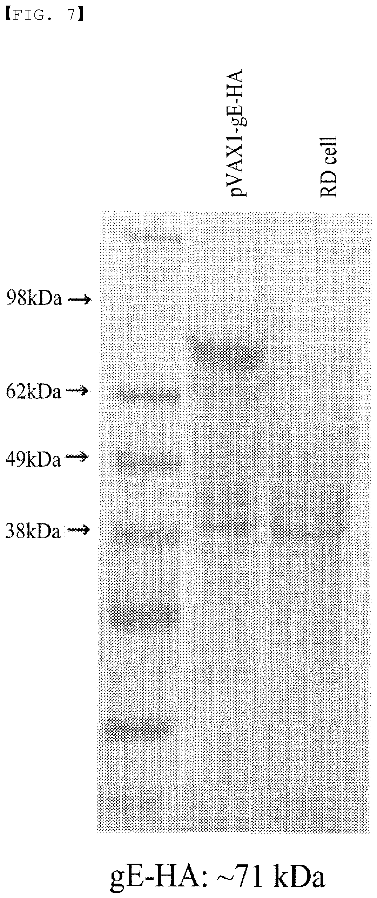

Referring to FIG. 7, the plasmid encoding a gE gene was introduced into RD cells derived from human rhabdomyosarcoma using Lipofectamine 2000. After 24 hours, the cells were lysed to extract proteins, and the proteins were then subjected to Western Blotting using an antibody for detecting an expressed antigen. An expressed protein of the plasmid encoding the gE gene was detected using HA-probe HRP (SantaCruz, Cat. No.: SC-805HRP), and visualized using a HRP chromogenic substrate (Invitrogen, Cat. No.: WP20004). As a result, it was revealed that the product size of the insert gene expressed from the plasmid encoding the gE gene was identical to the predicted molecular weight of the insert gene, indicating that the product of the gene introduced into the RD cells was the gE antigen protein of VZV.

## Claims

1. A plasmid DNA for preventing and treating herpes zoster, comprising an insertion site of a varicella-zoster virus (VZV)-derived gene encoding a VZV protein,
wherein the plasmid DNA is directly administered into the body by an electroporation method to induce immune activity of T cells against a VZV antigen.

2. The plasmid DNA of claim 1, wherein the gene comprises one selected from the group consisting of a first gene encoding an IE-62 protein (SEQ ID NO: 1), a second gene encoding an IE-63 protein (SEQ ID NO: 2), and a third gene encoding a gE protein (SEQ ID NO: 3).

3. The plasmid DNA of claim 1, which comprises one selected from the group consisting of a plasmid DNA set forth in SEQ ID NO: 4, a plasmid DNA set forth in SEQ ID NO: 5, and a plasmid DNA set forth in SEQ ID NO: 6.

4. A DNA vaccine composition for preventing and treating herpes zoster, comprising:
at least one plasmid containing an insertion site of a varicella-zoster virus (VZV)-derived gene encoding a VZV protein; and
other pharmaceutically acceptable ingredients.

5. The DNA vaccine composition of claim 4, wherein the plasmid comprises a plurality of plasmids containing different insertion sites of heterologous genes.

6. The DNA vaccine composition of claim 4, wherein the plasmid comprises:
a first plasmid containing an insertion site of a first gene encoding an IE-62 protein (SEQ ID NO: 1);
a second plasmid containing an insertion site of a second gene encoding an IE-63 protein (SEQ ID NO: 2); and
a third plasmid containing an insertion site of a third gene encoding a gE protein (SEQ ID NO: 3).

7. A method for activating T cells against a varicella-zoster virus (VZV) antigen, comprising:
preparing a plasmid containing a VZV-derived gene encoding a VZV protein; and
administering the plasmid into the body using an electroporation method.
